# EUROPÄISCHE PATENTANMELDUNG

(11) **EP 4 454 701 A1**
(43) Veröffentlichungstag der Anmeldung: **30.10.2024**
(21) Anmeldenummer: 23169563.6
(22) Anmeldetag: 24.04.2023
(51) Int. Cl.: A61N 5/06, A61N 5/067, A61N 1/36

(54) **VERFAHREN FÜR DEN BETRIEB EINES OPTISCHEN COCHLEA-IMPLANTATS UND OPTISCHES COCHLEA-IMPLANTAT ZUR DURCHFÜHRUNG DES VERFAHRENS**

(71) Anmelder: Georg-August-Universität Göttingen Stiftung Öffentlichen Rechts, Universitätsmedizin, 37075 Göttingen (DE); OptoGenTech GmbH, 37073 Göttingen (DE)
(72) Erfinder: Moser, Tobias, 37079 Göttingen (DE); Jablonski, Lukasz, 37075 Göttingen (DE); Goßler, Christian, 37077 Göttingen (DE)
(74) Vertreter: REHBERG HÜPPE + PARTNER

(57) **Zusammenfassung**

Beim Betreiben eines optischen Cochlea-Implantats mit mehreren Laserdioden (7) werden die Laserdioden (7), die verschiedenen Schallfrequenzen zugeordnet sind, abhängig von einem Signal angesteuert, das ein Schallfrequenzspektrum beschreibt, um Laserlicht (10) zu erzeugen und in verschiedene Lichtleiter (11) einzukoppeln. Die Lichtleiter (11) sind dazu vorgesehen, das Laserlicht (10) zu verschiedenen lichtempfindlichen Bereichen (24) einer Cochlea (23) zu leiten. Die Laserdioden (7) werden jeweils in mehreren voneinander getrennten Pulselets (19), die einzeln keine neurale Aktivierung der lichtempfindlichen Bereiche (24) der Cochlea (23) zur Folge haben, angesteuert. Dabei betragen Tastgrade der Pulselets (19) nicht mehr als 25 %; und die Pulselets (19), in denen verschiedene der Laserdioden (7) angesteuert werden, werden zeitlich so ineinander geschachtelt, dass nicht mehr als ein Viertel der Laserdioden (7) gleichzeitig angesteuert wird.

## Beschreibung

### TECHNISCHES GEBIET DER ERFINDUNG

Die Erfindung bezieht sich auf ein Verfahren für den Betrieb eines optischen Cochlea-Implantats und auf ein optisches Cochlea-Implantat zur Durchführung des Verfahrens. Insbesondere bezieht sich die Erfindung auf ein Verfahren mit den Merkmalen des Oberbegriffs des Patentanspruchs 1 und auf ein optisches Cochlea-Implantat mit den Merkmalen des Oberbegriffs des Patentanspruchs 9.

### STAND DER TECHNIK

Die WO 2017 / 011 396 A1 beschreibt Verfahren zum frequenzmodulierten Phasenkodieren (Frequency-Modulated Phase Coding = FMPC) für elektrische Cochlea-Implantate und Cochlea-Implantate, die diese Verfahren umsetzen. Ein ein Schallfrequenzspektrum beschreibendes Signal wird in mehrere Frequenzbänder aufgeteilt und jedem Frequenzband werden unterschiedliche Sammelbehälter zugeordnet, die jeweils einem Energieniveau des Frequenzspektrums innerhalb des jeweiligen Frequenzbands innerhalb eines Zeitraums zugeordnet sind. Für jeden Sammelbehälter wird ein Puls in einer Elektrode erzeugt, die dem Frequenzband zugeordnet ist, welches dem Sammelbehälter zugeordnet ist, wenn bestimmte Voraussetzungen in Bezug auf den Sammelbehälter erfüllt sind. Jedes Frequenzband entspricht einem Kanal und jedem Frequenzband ist eine Elektrode eines Elektrodenarrays zugeordnet. Das bekannte Verfahren kodiert akustische Informationen in elektrische Pulse. Dabei kann die Stimulation gleichzeitig an allen Elektroden des Elektrodenarrays erfolgen, wobei die Anzahl der unabhängigen Kanäle bis zu 22 betragen kann. Die mittleren Pulswiederholungsraten in jedem Kanal betragen ungefähr 100 bis 300 Hz. Lautstärke wird durch die Anzahl der Pulse in jedem Kanal und die Gesamtzahl der Pulse über die Kanäle kodiert und erfordert keine Erhöhung des Stroms in jedem Kanal. Die niedrige mittlere Pulswiederholungsrate für jeden Kanal und die Tatsache, dass Lautstärkeanstieg nicht durch einen Anstieg bei der Stromamplitude kodiert wird, reduzieren den Energieverbrauch der Cochlea-Implantate um einen Faktor von ungefähr 5 bis 10. Praktisch wird das akustische Signal mit einem Mikrofon empfangen. Eine Reihe von Filtern extrahiert die akustische Information in die Frequenzbänder. Auf jedes Frequenzband wird eine Kurzzeit-Fourier-Transformation angewendet, um die Einhüllende des Zeitsignals zu extrahieren. Ein Pulsgenerator erzeugt für jedes Frequenzband Folgen von Pulsen mit zufälligen Zeiträumen zwischen den Pulsen, während die mittlere Pulsrate variiert wird und proportional zu der Amplitude der extrahierten Einhüllenden ist.

FU-YU BEVERLY CHEN ET AL.: "Pulse-Width Modulation of Optogenetic Photo-Stimulation Intensity for Application to Full-Implantable Light Sources", IEEE Transactions on Biomedical Circuits and Systems, Band 11, Ausgabe 1, Februar 2017, Seiten 28-34 offenbaren eine optogenetische Stimulation von Neuronen durch eine gepulste Laserdiode. Die Laserdiode in Pulsen angesteuert, die einen variablen Tastgrad von beispielsweise 50 % oder 70 % und eine Pulsfolgefrequenz von 1 kHz bis 1.000 kHz aufweisen. Die Dauer der gepulsten Anregung liegt im Bereich von 4 ms.

DANIEL A TAFT ET AL.: "Across-frequency delays based on the cochlear traveling wave: enhanced speech presentation for cochlear implants", IEEE Trans Biomed Eng., März 2010, 57(3):596-606 beschreiben die Stimulation eines Hörnervs mit den Ausgängen einer Bank von schmalbandigen Filtern in elektrischen Cochlea-Implantaten. Um das Verstehen von Sprache zu verbessern, schlagen sie eine Desynchronisation der Frequenzbänder wie in einer normalen Cochlea vor. In einer normalen Cochlea werden die näher an der Cochlea-Basis liegenden und den höheren Tonfrequenzen entsprechenden Neuronen später aktiviert als diejenigen, die näher an der Cochlea-Spitze liegen und niedrigeren Tonfrequenzen entsprechen. Durch das zeitliche Versetzen der Frequenzbänder zueinander soll Sprache besser verstanden werden, weil bei der selektiven Wahrnehmung von Maxima weniger Stimulationen unbeachtet bleiben.

Aus BARBARA K. MÜLLER ET AL.: "Pulsed Interleaved Excitation", Biophysical Journal, Research Article, Band 89, Ausgabe 5, Seiten 3508-3522, November 2005 ist ein Verfahren zur gepulsten Anregung verschiedener Fluorophore einer Probe beim mehrfarbigen Fluoreszenzabbilden, in der Fluoreszenzkreuzkorrelationsspektroskopie (Fluorescence Cross-Correlation Spectroscopy = FCCS) und in Messungen des Einzelpaarfluoreszenzresonanzenergietransfers (single-pair Fluorescence Resonance Energy Transfer = spFRET) bekannt. Pulse von verschiedenen Anregungslichtquellen, die bei unterschiedlichen Wellenlängen emittierten, werden zeitlich so ineinander geschachtelt, dass die durch einen Puls erzeugte Fluoreszenzemission abgeschlossen ist, bevor der nächste Anregungspuls ankommt. Dadurch ist die Anregungsquelle für jedes detektierte Photon durch zeitliche Kodierung bekannt.

Bei der Entwicklung von optischen Cochlea-Implantaten stellt sich heraus, dass dann, wenn dieselben zeitlichen Anregungsmuster angewandt werden, wie bei bekannten elektrischen Cochlea-Implantaten ein signifikant höherer Bedarf an elektrischer Leistung auftritt. Der Bedarf an elektrischer Leistung ist dann besonders hoch, wenn mit dem optischen Cochlea-Implantat abhängig von dem Schallfrequenzspektrum eines zu übermittelnden Signals unterschiedliche Bereiche der Cochlea und damit potenziell mehrere Bereiche der Cochlea gleichzeitig neural aktiviert werden sollen.

### AUFGABE DER ERFINDUNG

Der Erfindung liegt die Aufgabe zugrunde, ein Verfahren zum Betreiben eines optischen Cochlea-Implantats aufzuzeigen, mit dem die Leistungsaufnahme beim gleichzeitigen neuralen Aktivieren verschiedener Bereiche der Cochlea begrenzt wird, so dass sehr viele Bereiche der Cochlea parallel zueinander und damit potenziell auch gleichzeitig neural aktiviert werden können, ohne eine Energiequelle des Cochlea-Implantats zu überlasten.

Neben einem optischen Cochlea-Implantat zu Durchführung dieses Verfahrens soll ein optisches Cochlea-Implantat aufgezeigt werden, das trotz einer Vielzahl von Laserdioden und Lichtleitern zur neuralen Aktivierung einer Vielzahl verschiedener lichtempfindlicher Bereiche einer Cochlea einen besonders kompakten Aufbau aufweist.

### LÖSUNG

Die Aufgaben der Erfindung werden durch ein Verfahren mit den Merkmalen des Patentanspruchs 1 und durch optische Cochlea-Implantate mit den Merkmalen des Patentanspruchs 9 bzw. mit den Merkmalen des Patentanspruchs 11 gelöst. Bevorzugte Ausgestaltungen des Verfahrens und der optischen Cochlea-Implantate sind den abhängigen Patentansprüchen zu entnehmen.

### BESCHREIBUNG DER ERFINDUNG

Bei einem erfindungsgemäßen Verfahren zum Betreiben eines optischen Cochlea-Implantats mit mehreren Laserdioden werden die verschiedenen Schallfrequenzen zugeordneten Laserdioden abhängig von einem ein Schallfrequenzspektrum beschreibenden Signal angesteuert, um Laserlicht zu erzeugen und in verschiedene Lichtleiter einzukoppeln. Die Lichtleiter sind dazu vorgesehen, das Laserlicht zu verschiedenen lichtempfindlichen Bereichen einer Cochlea zu leiten. Die Laserdioden werden jeweils in mehreren voneinander getrennten Pulselets, die einzeln keine neurale Aktivierung der lichtempfindlichen Bereiche der Cochlea zur Folge haben, angesteuert. Dabei betragen die Tastgrade der Pulselets nicht mehr als 25 %, und die Pulselets, in denen verschiedene der Laserdioden angesteuert werden, werden zeitlich so ineinander geschachtelt, dass nicht mehr als ein Viertel der Gesamtheit der Laserdioden gleichzeitig angesteuert wird.

Es versteht sich, dass das Schallfrequenzspektrum und das das Schallfrequenzspektrum beschreibende Signal zeitvariabel sind und ihre Variabilität über der Zeit einen wesentlichen Teil der Information kodiert, die mit Hilfe des Cochlea-Implantats auf die Cochlea übertragen wird. Die Übertragung der Information erfolgt durch neurale Aktivierung der lichtempfindlich gemachten Bereiche der Cochlea in unterschiedlichen Kombinationen und zeitlichen Abfolgen. Die neurale Aktivierung erfolgt durch Laserlicht von den Laserdioden, die verschiedenen Schallfrequenzen zugeordnet sind. Dabei wird jede einzelne neurale Aktivierung eines der lichtempfindlichen Bereiche der Cochlea nicht durch Ansteuern der jeweiligen Laserdiode in einem einzelnen geschlossenen Puls, so dass die Laserdiode einen einzelnen geschlossenen Lichtpuls des Laserlichts ausgibt, bewirkt. Stattdessen erfolgt die Ansteuerung der jeweiligen Laserdiode für die jeweilige neurale Aktivierung durch eine Folge von mehreren voneinander getrennten Pulselets, die einzeln keine neurale Aktivierung des lichtempfindlichen Bereichs der Cochlea zur Folge hätten, die aber in ihrer Gesamtheit die gewünschte neurale Aktivierung bewirken. Dass zur neuralen Aktivierung eines lichtempfindlichen Bereichs der Cochlea kein geschlossener Lichtpuls erforderlich ist, sondern eine Folge von Pulselets vergleichbarer Lichtintensität und damit reduzierter Gesamtlichtenergie und elektrischer Energie zur Ansteuerung der jeweiligen Laserdiode ausreichend ist, ist grundsätzlich bekannt. Die vorliegende Erfindung nutzt dieses Konzept als Ausgangspunkt und wendet es mit besonders niedrigen Tastgraden der Pulselets von nicht mehr als 25 % an. Zusätzlich werden die Pulselets, in denen verschiedene der Laserdioden zeitlich überlappend angesteuert werden, so ineinander geschachtelt, dass die Gesamtzahl der gleichzeitig angesteuerten Laserdioden und damit die für die Ansteuerung benötigte elektrische Gesamtleistung begrenzt wird. So wird ein Energiespeicher des Cochlea-Implantats selbst dann nicht überlastet, wenn das Signal ein Geräusch mit hoher Lautstärke in allen Frequenzbereichen, das auch als lautes weißes Rauschen bezeichnet werden kann, kodiert. Durch den maximalen Tastgrad der Pulselets von nicht mehr als 25 % können die Pulselets von mindestens vier zeitlich überlappend angesteuerten Laserdioden so ineinander geschachtelt werden, dass die Amplitude der hierfür benötigten elektrischen Leistung nicht größer ist als beim Ansteuern einer einzelnen Laserdiode. Bezogen auf die Gesamtheit der Laserdioden werden die Pulselets so ineinander geschachtelt, dass nicht mehr als ein Viertel der Laserdioden gleichzeitig - also mit zeitgleichen Pulselets - angesteuert wird. Dadurch wird die Amplitude der elektrischen Leistung beim Ansteuern der Laserdioden von der maximal möglichen Amplitude beim gleichzeitigen Ansteuern aller Laserdioden auf ein Viertel reduziert.

Die Pulseletfolgefrequenz der Pulselets, in denen eine einzelne der Laserdioden angesteuert wird, liegt in der Regel zwischen 5 kHz und 200 MHz und vielfach zwischen 10 kHz und 100 MHz. Oft liegt die Obergrenze der Pulseletfolgefrequenz bei 10 MHz oder sogar bei nur 1 MHz. Bei Verfügbarkeit entsprechender Laserdioden und Treiber kann die Pulseletfolgefrequenz aber auch noch höher als 200 MHz sein. Es versteht sich, dass die Pulseletfolgefrequenzen aller Pulselets, die zeitlich ineinander geschachtelt werden, um mehrere der Laserdioden zeitlich überlappend anzusteuern, gleich sind oder ganzzahlige Vielfache voneinander sind.

Durch weitere Reduktion der maximalen Tastgrade der Pulselets auf nicht mehr als 12,5 % kann die Anzahl der zeitlich überlappend ansteuerbaren Laserdioden, ohne dass die Amplitude der elektrischen Leistung gegenüber der Amplitude beim Ansteuern nur einer einzigen Laserdiode ansteigen muss, auf acht erhöht werden.

Bei dem erfindungsgemäßen Verfahren kann mit Hilfe der Tastgrade der Pulselets die Lautstärke bei der der jeweiligen Laserdiode zugeordneten Schallfrequenz kodiert werden. Je höher die Lautstärke und damit die Amplitude des Signals bei der jeweiligen Schallfrequenz ist, desto größer ist der Tastgrad, der jedoch den für alle Laserdioden geltenden maximalen Tastgrad nicht überschreiten darf.

Um die Information zu übermitteln, dass das Schallfrequenzspektrum bei einer Schallfrequenz eine besonders hohe Amplitude aufweist, d. h. bei dieser Schallfrequenz besonders laut ist, können ab einer Mindestamplitude des Signals bei dieser Schallfrequenz neben der Laserdiode, die dieser Schallfrequenz zugeordnet ist, auch solche der Laserdioden angesteuert werden, die benachbarten Schallfrequenzen zugeordnet sind. Dadurch wird das Laserlicht auch in solche der Lichtleiter eingekoppelt, die dazu vorgesehen sind, das Laserlicht zu benachbarten lichtempfindlichen Bereichen der Cochlea zu leiten. Bis zu der Mindestamplitude des Signals bei der jeweiligen Schallfrequenz kann die Amplitude allein durch den Tastgrad der Pulselets kodiert werden, in denen die zugehörige Laserdiode angesteuert wird.

Bei dem erfindungsgemäßen Verfahren können die Pulselets, in denen eine der Laserdioden angesteuert wird, nacheinander mit den Pulselets geschachtelt werden, in denen verschiedene andere der Laserdioden angesteuert werden. D. h., die Auswahl der Laserdioden, die zeitlich überlappend angesteuert werden, kann variieren, und zwar auch über eine einzelne neurale Aktivierung eines lichtempfindlichen Bereichs der Cochlea hinweg. Das nacheinander Schachteln der Pulselets zum zeitlich überlappenden Ansteuern wechselnder Teilmengen der Laserdioden kann damit kombiniert werden, dass die Laserdioden abhängig von dem das Schallfrequenzspektrum beschreibenden Signal in einer Reihenfolge der Schallfrequenzen angesteuert werden, denen die einzelnen Laserdioden zugeordnet sind. Dabei können die Schallfrequenzen in Richtung der Reihenfolge abfallen, d. h. zunächst die Laserdioden angesteuert werden, die höheren Schallfrequenzen entsprechen, bevor Laserdioden angesteuert werden, die niedrigeren Schallfrequenzen entsprechen. Dies ist die Reihenfolge, in der eine normale Cochlea verschiedene Schallfrequenzen sequentiell, d. h. mit kleinem Zeitversatz wahrnimmt.

Dass die Laserdioden abhängig von dem Signal in einer Reihenfolge ansteigender Schallfrequenzen angesteuert werden, dreht die aus dem räumlichen Aufbau einer Cochlea resultierende Abfolge um, mit der eintretender Schall zu einer neuralen Aktivierung führt. Bei einer Cochlea liegen die Bereiche, die für Schall höherer Frequenzen empfindlich sind, weiter außen und werden daher normalerweise eher neural aktiviert.

Vorzugsweise werden bei dem erfindungsgemäßen Verfahren die Pulselets in voneinander getrennten Pulseletgruppen gruppiert, die jeweils einen von voneinander getrennten effektiven Pulsen bilden. Jeder der effektiven Pulse kann eine neurale Aktivierung eines der lichtempfindlichen Bereiche der Cochlea bewirken. Eine Pulsfolgefrequenz der effektiven Pulse liegt im Bereich von 50 Hz bis 1.000 Hz. Ein Tastgrad der effektiven Pulse beträgt nicht mehr als 50 %, vorzugsweise nicht mehr als 25 %. Der Kehrwert der Pulsfolgefrequenz bestimmt die zeitliche Auflösung, mit der das Schallfrequenzspektrum durch die Pulse kodiert wird.

Vorzugsweise werden bei dem erfindungsgemäßen Verfahren jeweils nicht mehr als vier und mehr bevorzugt jeweils nicht mehr als zwei der Laserdioden gleichzeitig angesteuert. Damit wird die Amplitude der elektrischen Leistung, die für das Ansteuern der Laserdioden benötigt wird, auf das Vierfache bzw. Zweifache der Amplitude der elektrischen Leistung beim Ansteuern einer einzelnen Laserdiode reduziert. Diese Beschränkung der Anzahl der gleichzeitig angesteuerten Laserdioden kann mit einem Konzept kombiniert werden, bei dem Diodengruppen von jeweils einigen der Laserdioden getrennt voneinander angesteuert werden, wobei vorzugsweise nicht mehr als eine einzige der Laserdioden jeder Diodengruppe gleichzeitig angesteuert wird. Konkret können die Laserdioden beispielsweise in zwei gleichgroße Diodengruppen unterteilt werden, wobei jede Diodengruppe beispielsweise 16 und vorzugsweise 32 Laserdioden umfasst. So können Laserdioden zum separaten neuralen Aktivieren von 32 bzw. 64 lichtempfindlichen Bereichen der Cochlea angesteuert werden.

Vorzugsweise werden die Laserdioden in den Pulselets jeweils mit einem elektrischen Strom von mindestens 25 mA, noch mehr bevorzugt von mindestens 50 mA und am meisten bevorzugt von mindestens 75 mA angesteuert. Alternativ oder zusätzlich können die Laserdioden in den Pulselets jeweils mit einem elektrischen Strom von mindestens 80 % des Nennstroms der jeweiligen Laserdiode angesteuert werden. Die Ansteuerung kann auch mit einem elektrischen Strom von mehr als 100 % des Nennstroms der jeweiligen Laserdiode erfolgen, beispielsweise mit einem elektrischen Strom von bis zu 120 % oder sogar von bis zu 150% des Nennstroms. Die relative Lichtleistung von Laserdioden steigt mit dem Strom, mit dem die Laserdioden angesteuert werden, an. Dies gilt auch für Ströme oberhalb der Nennströme, mit denen die Laserdioden im Dauerstrichbetrieb angesteuert werden können, ohne sie zu zerstören. Wenn eine Laserdiode - statt mit einem konstanten Strom - in Pulsen mit derselben mittleren elektrischen Leistung angesteuert wird, ergibt sich daher eine höhere Lichtausbeute, d.h. eine höhere mittlere Lichtleistung des Laserlichts von der Laserdiode. Da Laserdioden kurzzeitig mit hohen Strömen angesteuert werden können, ohne sie zu beschädigen, kann auch auf diese Weise der für bestimmte neurale Aktivierungen benötigte Bedarf des Cochlea-Implantats an elektrischer Energie reduziert werden. Vorzugsweise werden die Laserdioden bei dem erfindungsgemäßen Verfahren also mit Strömen angesteuert, die oberhalb ihres Nennstroms liegen, mit dem sie im Dauerstrichbetrieb angesteuert werden können.

Ein erfindungsgemäßes optisches Cochlea-Implantat zur Durchführung des erfindungsgemäßen Verfahrens weist mehrere Laserdioden, mehrere Lichtleiter, die dazu angeordnet und ausgebildet sind, Laserlicht von jeweils einer der Laserdioden in einen von verschiedenen lichtempfindlichen Bereichen einer Cochlea zu leiten, einen Energiespeicher und eine Steuerung auf. Die Steuerung steuert die verschiedenen Schallfrequenzen zugeordneten Laserdioden mit elektrischen Strömen aus dem Energiespeicher an, um das Laserlicht zu erzeugen und in die Lichtleiter einzukoppeln. Das Ansteuern erfolgt abhängig von einem ein Schallfrequenzspektrum beschreibenden Signal. Die Steuerung ist erfindungsgemäß dazu ausgebildet, die Laserdioden gemäß dem erfindungsgemäßen Verfahren anzusteuern.

Einen wesentlichen Einfluss auf den Energie- und Leistungshaushalt eines optischen Cochlea-Implantats hat die Effizienz, mit der das Laserlicht von den Laserdioden unter den Randbedingungen der Implantierfähigkeit des Cochlea-Implantats in die Lichtleiter eingekoppelt wird. Zu diesen Randbedingungen zählt es, dass die Laserdioden und die daran angeschlossenen elektrischen Treiber nicht nur elektrisch zu isolieren, sondern hermetisch abzuschirmen sind. Die dafür vorzusehende Abschirmung kann ein Fenster aufweisen, durch das hindurch das Laserlicht in die Lichtleiter eingekoppelt wird. Dabei können größere Verluste an Laserlicht, das nicht mehr zur neuralen Aktivierung der Cochlea zur Verfügung steht, vermieden werden, indem zwischen den Laserdioden und dem Fenster über das hinweg sich zumindest einige der Laserdioden und Eintrittsquerschnitte der zugehörigen Lichtleiter gegenüberliegen, ein Mikrolinsenarray angeordnet ist, dessen Mikrolinsen das Laserlicht von jeweils einer der Laserdioden in den Eintrittsquerschnitt des zugehörigen Lichtleiters fokussieren. Besonders effektiv ist die Einkopplung, wenn optische Weglängen zwischen den Austrittflächen der Laserdioden und den Eintrittsquerschnitten der zugehörigen Lichtleiter auf nicht mehr als 1,5 mm begrenzt werden.

Ein anderes erfindungsgemäßes optisches Cochlea-Implantat weist mehrere Laserdioden, mehrere Lichtleiter, die dazu angeordnet und ausgebildet sind, Laserlicht von jeweils einer der Laserdioden in einen von verschiedenen lichtempfindlichen Bereichen einer Cochlea zu leiten, und ein optisches Fenster auf, über das hinweg sich zumindest einige der Laserdioden und Eintrittsquerschnitte der zugehörigen Lichtleiter gegenüberliegen. Zwischen den Laserdioden und dem Fenster ist ein Mikrolinsenarray angeordnet, dessen Mikrolinsen das Laserlicht von jeweils einer der Laserdioden in den Eintrittsquerschnitt des zugehörigen Lichtleiters fokussieren, und optische Weglängen zwischen den Austrittsflächen der Laserdioden und den Eintrittsquerschnitten der zugehörigen Lichtleiter sind nicht länger als 1,5 mm. Hiermit wird eine hohe Effizienz der Einkopplung des Laserlichts von den Laserdioden in die Lichtleiter und damit eine Begrenzung der nötigen elektrischen Leistung bei der Ansteuerung der Laserdioden erreicht, und zwar ganz unabhängig davon wie diese elektrische Ansteuerung der Laserdioden erfolgt.

Bei den erfindungsgemäßen optischen Cochlea-Implantaten mit dem Mikrolinsenarray vor dem Fenster weist das optische Fenster vorzugsweise eine Dicke von 0,2 mm bis 0,6 mm, noch mehr bevorzugt von 0,3 mm bis 0,4 mm und damit etwa 0,35 mm auf. Weiterhin ist es bevorzugt, wenn das Fenster aus Saphir ausgebildet ist. Saphir ist biokompatibel, sehr beständig und gut mit einem Gehäuse des Cochlea-Implantats aus Titan kombinierbar. Das Mikrolinsenarray ist vorzugsweise mit einem beidseitig mikrostrukturierten Wafer ausgebildet. Dabei kann der Wafer im Bereich zumindest einiger der Mikrolinsen auf zumindest einer seiner Seiten asphärisch strukturiert sein, um die gewünschte Fokussierung des Laserlichts von den Laserdioden in die Eintrittsquerschnitte der Lichtleiter möglichst perfekt zu bewirken.

Die Laserdioden können vor dem optischen Fenster in kleinen seitlichen Abständen von 70 µm bis 150 µm angeordnet sein. Vorzugsweise liegen die seitlichen Abstände der Laserdioden in einem Bereich von 90 µm bis 110 µm, d. h. bei 100 µm.

Eine der jeweiligen Laserdiode zugekehrte erste Linsenfläche des Mikrolinsenarrays kann einen Krümmungsradius im Bereich von 0,060 mm bis 0,075 mm oder genau von 0,0687 mm aufweisen. Eine konische Konstante der jeweiligen Linsenfläche kann im Bereich von -2,60 bis -2,50 liegen oder genau -2,547 betragen. Eine aus der ersten Linsenfläche resultierende Brennweite kann im Bereich von 0,14 mm bis 0,16 mm oder genau bei 0,150 mm liegen. Eine der jeweiligen Laserdiode abgekehrte zweite Linsenfläche des Mikrolinsenarrays kann einen Krümmungsradius von 0,120 mm bis 0,130 mm oder genau von 0,1264 mm, eine konische Konstante von -0,50 bis -0,40 oder genau von -0,450 und eine Brennweite von 0,35 mm bis 0,50 mm oder genau von 0,433 mm aufweisen. Die angegebenen genauen Werte sind diejenigen eines konkreten Ausführungsbeispiels des erfindungsgemäßen optischen Cochlea-Implantats, welches mit Erfolg praktisch erprobt wurde.

Ein Arbeitsabstand der jeweiligen Laserdiode zu der jeweiligen Mikrolinse kann dann zwischen 0,14 mm und 0,16 mm oder genau 0,150 mm betragen. Eine Linsendicke der jeweiligen Mikrolinse kann zwischen 0,35 mm bis 0,45 mm oder genau 0,410 mm betragen. Ein Arbeitsabstand der jeweiligen Mikrolinse zu dem optischen Fenster ist vorzugsweise nicht größer als 0,400 mm und beträgt vorzugsweise zwischen 0,09 mm und 0,11 mm oder genau 0,100 mm; und ein Arbeitsabstand des optischen Fensters zu den Lichtleitern ist vorzugsweise nicht größer als 0,200 mm und beträgt vorzugsweise zwischen 0,09 mm und 0,11 mm oder genau 0,100 mm. Der Arbeitsabstand des optischen Fensters zu den Lichtleitern kann dabei mit transparentem Epoxidharz überbrückt sein, in das die Lichtleiter eingebettet sind und das die Lichtleiter gegenüber dem optischen Fenster fixiert. Die optische Weglängen zwischen den Austrittsflächen der Laserdioden und den Eintrittsquerschnitten der zugehörigen Lichtleiter, die aus den voranstehenden genauen Werten des konkreten Ausführungsbeispiels des erfindungsgemäßen optischen Cochlea-Implantats resultieren, betragen 0,15 mm + 0,41 mm + 0,10 mm + 0,35 mm + 0,10 mm = 1,11 mm.

Vorteilhafte Weiterbildungen der Erfindung ergeben sich aus den Patentansprüchen, der Beschreibung und den Zeichnungen.

Die in der Beschreibung genannten Vorteile von Merkmalen und von Kombinationen mehrerer Merkmale sind lediglich beispielhaft und können alternativ oder kumulativ zur Wirkung kommen, ohne dass die Vorteile zwingend von erfindungsgemäßen Ausführungsformen erzielt werden müssen.

Hinsichtlich des Offenbarungsgehalts - nicht des Schutzbereichs - der ursprünglichen Anmeldungsunterlagen und des Patents gilt Folgendes: Weitere Merkmale sind den Zeichnungen - insbesondere den dargestellten Geometrien und den relativen Abmessungen mehrerer Bauteile zueinander sowie deren relativer Anordnung und Wirkverbindung - zu entnehmen. Die Kombination von Merkmalen unterschiedlicher Ausführungsformen der Erfindung oder von Merkmalen unterschiedlicher Patentansprüche ist ebenfalls abweichend von den gewählten Rückbeziehungen der Patentansprüche möglich und wird hiermit angeregt. Dies betrifft auch solche Merkmale, die in separaten Zeichnungen dargestellt sind oder bei deren Beschreibung genannt werden. Diese Merkmale können auch mit Merkmalen unterschiedlicher Patentansprüche kombiniert werden. Ebenso können in den Patentansprüchen aufgeführte Merkmale für weitere Ausführungsformen der Erfindung entfallen, was aber nicht für die unabhängigen Patentansprüche des erteilten Patents gilt.

Die in den Patentansprüchen und der Beschreibung genannten Merkmale sind bezüglich ihrer Anzahl so zu verstehen, dass genau diese Anzahl oder eine größere Anzahl als die genannte Anzahl vorhanden ist, ohne dass es einer expliziten Verwendung des Adverbs "mindestens" bedarf. Wenn also beispielsweise von einem Mikrolinsenarray die Rede ist, ist dies so zu verstehen, dass genau ein Mikrolinsenarray, zwei Mikrolinsenarrays oder mehr Mikrolinsenarrays vorhanden sind. Die in den Patentansprüchen angeführten Merkmale können durch weitere Merkmale ergänzt werden oder die einzigen Merkmale sein, die der Gegenstand des jeweiligen Patentanspruchs aufweist.

Die in den Patentansprüchen enthaltenen Bezugszeichen stellen keine Beschränkung des Umfangs der durch die Patentansprüche geschützten Gegenstände dar. Sie dienen lediglich dem Zweck, die Patentansprüche leichter verständlich zu machen.

### KURZBESCHREIBUNG DER FIGUREN

Im Folgenden wird die Erfindung anhand in den Figuren dargestellter bevorzugter Ausführungsbeispiele weiter erläutert und beschrieben.
- **Fig. 1**: zeigt die Anordnung von Laserdioden, Mikrolinsenarrays und Lichtleiter eines erfindungsgemäßen optischen Cochlea-Implantats.
- **Fig. 2**: zeigt die Anordnung gemäß Fig. 1 mit weiteren Bestandteilen eines Gehäuses des optischen Cochlea-Implantats.
- **Fig. 3**: zeigt ein Detail von Fig. 2, wobei zusätzlich ein optisches Fenster zwischen den Mikrolinsenarrays und den Lichtleitern dargestellt ist.
- **Fig. 4**: zeigt schematisch eine Pulseletgruppe von Pulselets, in denen eine der Laserdioden des optischen Cochlea-Implantats gemäß den Fig. 1 bis 3 angesteuert wird.
- **Fig. 5**: illustriert die zeitliche Abfolge der Ansteuerung mehrerer Laserdioden des optischen Cochlea-Implantats gemäß den Fig. 1 bis 3; und
- **Fig. 6**: zeigt schematisch die neurale Aktivierung unterschiedlicher lichtempfindlich gemachter Bereiche einer Cochlea mit Laserlicht von den Laserdioden des optischen Cochlea-Implantats gemäß den Fig. 1 bis 3.

### FIGURENBESCHREIBUNG

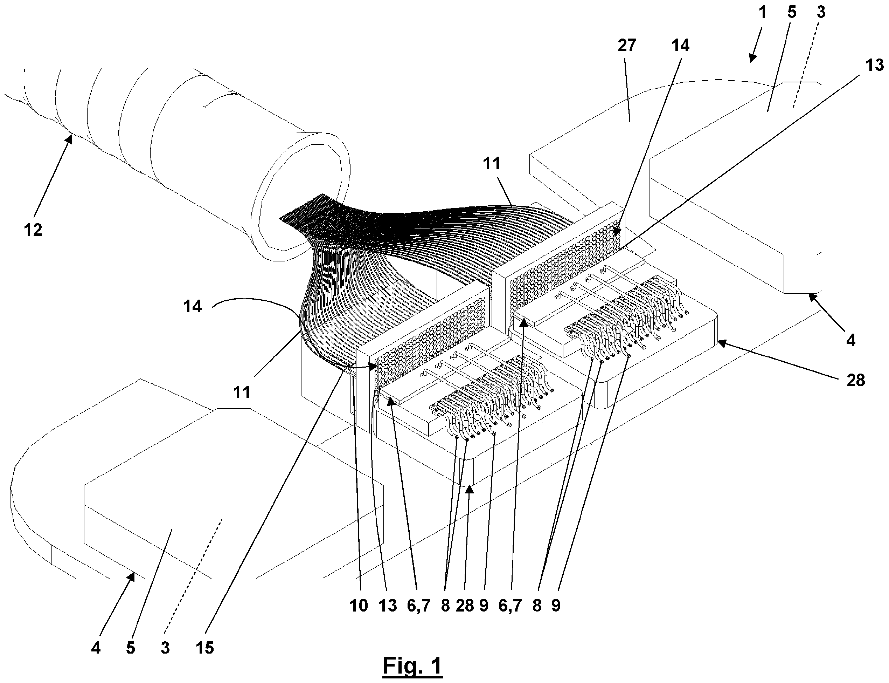

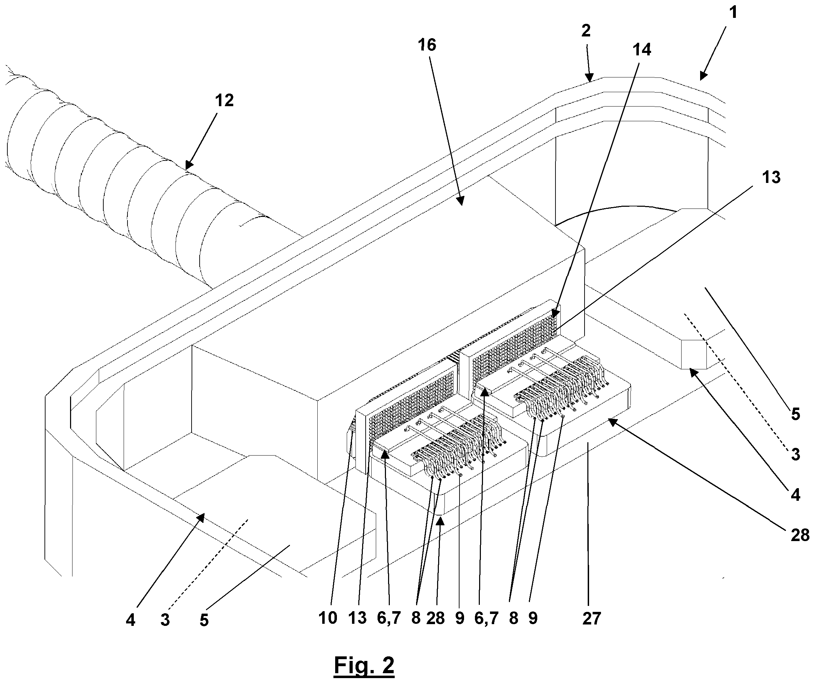

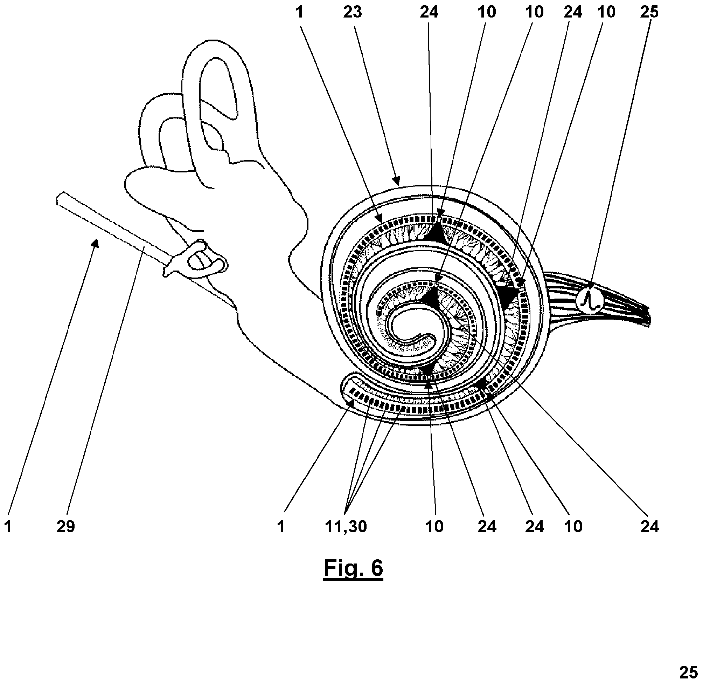

Die in **Fig. 1** dargestellten wesentlichen Teile eines optischen Cochlea-Implantats 1 sind in einem erst in Fig. 2 teilweise dargestellten Gehäuse 2 aus Titan angeordnet. In dem Gehäuse 2 ist auf einer Leiterplatte 27 eine Steuerung 4 mit integrierten Energiespeichern 3 für elektrische Energie montiert. Die Steuerung 4 weist hier zwei getrennte Treiber 5 für jeweils ein lineares Diodenarray 6 von 32 Laserdioden 7 auf. Die Diodenarrays 6 sind jeweils über einen Montageadapter 28 aus Keramik auf der Leiterplatte 27 montiert. Die Montageadapter 28 aus Keramik sind wichtig für das thermische Management der Laserdioden 7. Von jedem der Treiber 5 verlaufen 32 Steuerleitungen 8, an die jeweils eine der Laserdioden 7 angeschlossen ist, und vier Masseleitungen 9, an die jeweils acht der Laserdioden 7 angeschlossen sind, über einen der Montageadapter 28 zu dem zugehörigen Diodenarray 6. Laserlicht 10 von jeder einzelnen der Laserdioden 7 wird in einen von 64 Lichtleitern 11 eingekoppelt. Die Lichtleiter 11 münden in eine Schutzspirale 12 ein. Die Schutzspirale 12, die aus einem Metallband auf einen die Lichtleiter 11 aufnehmenden, in Fig. 6 gezeigten Silikonschlauch 29 gewickelt sein kann, verhindert ein Abknicken der aus dem Gehäuse 2 austretenden Lichtleiter 11. Die Einkopplung erfolgt jeweils über eine Mikrolinse 13 eines Mikrolinsenarrays 14. Das Mikrolinsenarray 14 ist ein zweidimensionales Mikrolinsenarray. Von den Mikrolinsen 13 des Mikrolinsenarrays 14 wird gemäß Fig. 1 jedoch nur eine einzelne Zeile von Mikrolinsen 13 genutzt. Das Mikrolinsenarray ist aus einem beidseitig mikrostrukturierten Wafer ausgebildet. Der Wafer ist im Bereich der Mikrolinsen 13 auf zumindest einer seiner Seiten asphärisch strukturiert, um das Laserlicht 10 möglichst perfekt in Eintrittsquerschnitte 15 der Lichtleiter 11 zu fokussieren. Ein zwischen den Mikrolinsenarrays 14 und den Eintrittsquerschnitten 15 der Lichtleiter 11 angeordnetes optisches Fenster ist in Fig. 1 nicht dargestellt. Die Laserdioden 7 sind verschiedenen Schallfrequenzen zugeordnet und werden abhängig von einem ein Schallfrequenzspektrum beschreibenden Signal angesteuert, um das Laserlicht 10 in die verschiedenen Lichtleiter 11 einzukoppeln. Die Lichtleiter 11 sind vorgesehen, das Laserlicht zu verschiedenen lichtempfindlichen Bereichen einer Cochlea zu leiten.

**Fig. 2** zeigt zusätzlich zu Fig. 1 einen Anschlussbereich 16 des Gehäuses 2, innerhalb dessen die hier nicht sichtbaren Lichtleiter 11 in transparentes Epoxidharz eingebettet sind, um sie gegenüber dem Gehäuse 2 und damit auch gegenüber dem auch hier nicht dargestellten optischen Fenster so zu fixieren, dass ihre Eintrittsquerschnitte 15 in der Brennpunkten der Mikrolinsen 13 liegen.

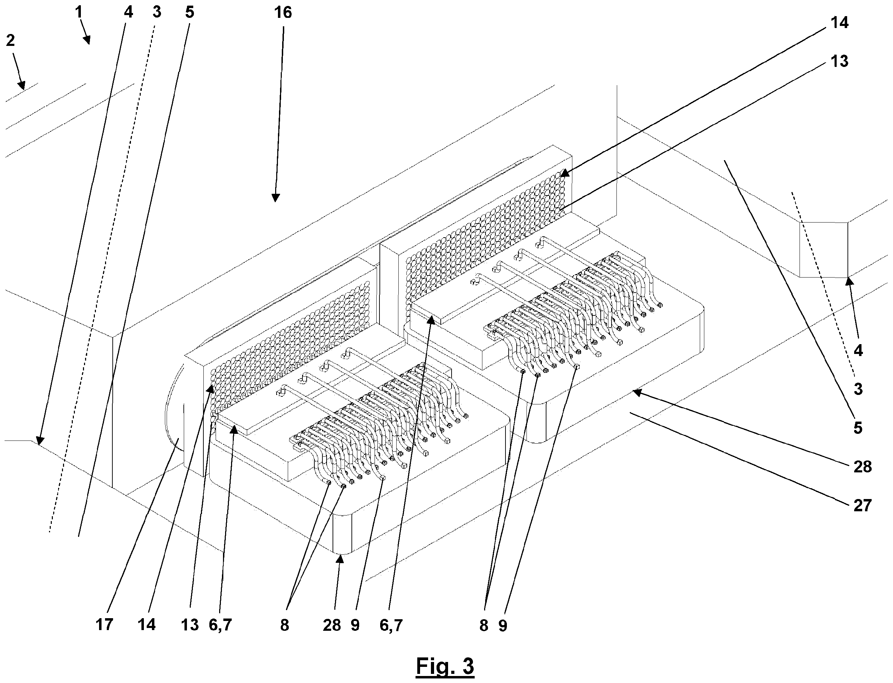

**Fig. 3** zeigt ein Detail von Fig. 2, bei dem jetzt das optische Fenster 17 zwischen den Eintrittsquerschnitten 15 der Lichtleiter 11 gemäß Fig. 1 und den Mikrolinsenarrays 14 wiedergegeben ist.

Weitere Details des konkreten Ausführungsbeispiels des optischen Cochlea-Implantats 1 gemäß den Figuren 1 bis 3, welches mit Erfolg praktisch erprobt wurde, sind oben in der Beschreibung der Erfindung angegeben.

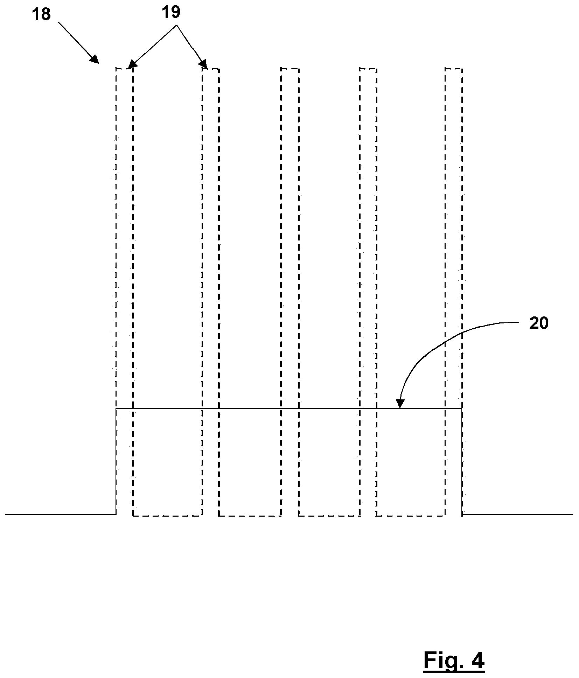

**Fig. 4** zeigt mit gestrichelter Linie eine Pulseletgruppe 18 von einzelnen Pulselets 19 gleichbleibender Amplitude und einem Tastgrad von etwa 20 %. Mit durchgezogener Linie ist ein Puls 20 mit einer Pulsenergie dargestellt, die genau so groß ist wie die Summe der Pulsenergien aller fünf Pulselets 19 der Pulseletgruppe 18. Entsprechend ist die konstante Amplitude des Pulses 20 nur ein Fünftel so groß wie die Amplitude der einzelnen Pulselets 19. Wenn die Laserdioden 7 statt in einem Puls 20 durch die Pulselets 19 der Pulseletgruppe 18 angesteuert werden, ist das Verhältnis zwischen den Lichtintensitäten des Laserlichts während der Pulselets 19 und während des Pulses 20 noch größer, weil die relative Lichtleistung von Laserdioden mit dem Strom, mit dem die Laserdioden angesteuert werden, ansteigt. Zudem steigt die Wirkung der neuralen Aktivierung von lichtempfindlichen Nervenzellen mit der zeitlichen Konzentration der Lichtenergie auf einzelne Lichtpulse an, selbst wenn die einzelnen Lichtpulse für die neurale Aktivierung nicht ausreichend sind, d. h. die gewünschte neurale Aktivierung nicht durch jedes einzelne Pulselet 19, sondern nur durch die gesamte Pulseletgruppe 18 erreicht wird. Die Ansteuerung der Laserdioden 7 des optischen Cochlea-Implantats 1 in in Pulseletgruppen 18 angeordneten Pulselets 19 weist daher mehrere energetische Vorteile auf.

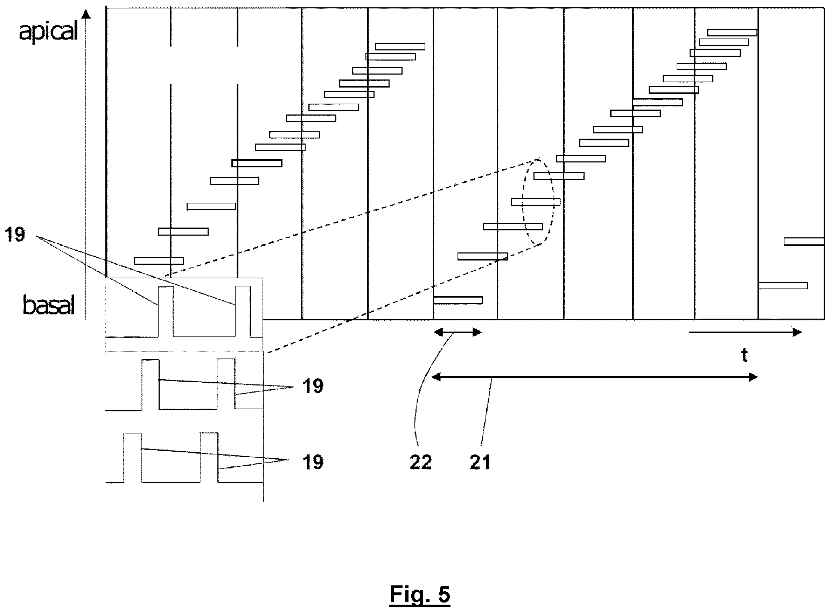

Zusätzlich werden bei dem optischen Cochlea-Implantat 1 die Pulselets 19, in denen verschiedene der Laserdioden 7 angesteuert werden, zeitlich ineinander geschachtelt, wie dies beispielhaft in **Fig. 5** illustriert ist. In Fig. 5 sind über der Zeit t aufeinanderfolgende Stimulationsperioden 21 dargestellt, in denen verschiedene Laserdioden 7 gemäß den Figuren 1 bis 3 jeweils während einer Pulsdauer 22 in Pulselets 19 einer Pulseletgruppe 18 angesteuert werden. Dabei sind die den einzelnen Laserdioden 7 zugeordneten Pulsdauern 22 so zueinander versetzt, dass die Laserdioden 7, die ihr Laserlicht 10 in die Lichtleiter 11 einkoppeln, welche zu näher an der Cochlea-Basis. d. h. basal, liegenden lichtempfindlichen Bereichen führen, eher angesteuert werden als die Laserdioden 7, die ihr Laserlicht 10 in die Lichtleiter 11 einkoppeln, welche zu näher an der Cochlea-Spitze, d. h. apical, liegenden lichtempfindlichen Bereichen der Cochlea führen. Soweit die Pulsdauern 22 dabei zeitlich überlappen, sind die einzelnen Pulselets 19 zeitlich so ineinander geschachtelt, dass von den hier betrachteten 16 Laserdioden niemals zwei gleichzeitig angesteuert werden. Separat dargestellt sind die Pulselets 19, in denen drei Laserdioden 7 angesteuert werden, die drei nebeneinander liegenden lichtempfindlichen Bereichen der Cochlea entsprechen. Die Pulselets 19 weisen einen Tastgrad von maximal 20 % auf, so dass auch fünf Laserdioden 7 mit ineinander geschachtelten Pulselets 19 quasi gleichzeitig angesteuert werden können, ohne dass zwei Pulselets 19 zeitlich überlappen.

**Fig. 6** zeigt eine Cochlea 23 mit spiralförmig angeordneten lichtempfindlichen Bereichen. Dabei ist angedeutet, dass einzelne lichtempfindliche Bereiche 24 durch das aus einem distalen Ende eines der Lichtleiter 11 des Cochlea-Implantat 1 austretende Laserlicht 10 von einer der Laserdioden 7 neural aktiviert werden, um die einem Schallfrequenzspektrum entsprechenden Informationen zu übermitteln. Die neuralen Aktivierungen resultieren in ein elektrisches Signal 25, das der Hörnerv 26 weiterleitet. Von dem Cochlea-Implantat 1 zeigt Fig. 6 den die Lichtleiter 11 umschließenden Silikonschlauch 29 und schematisch die distalen Enden 30 der einzelnen Lichtleiter 11.

### BEZUGSZEICHENLISTE

- 1: optisches Cochlea-Implantat
- 2: Gehäuse
- 3: Energiespeicher
- 4: Steuerung
- 5: Treiber
- 6: Diodenarray
- 7: Laserdiode
- 8: Steuerleitung
- 9: Masseleitung
- 10: Laserlicht
- 11: Lichtleiter
- 12: Schutzspirale
- 13: Mikrolinse
- 14: Mikrolinsenarray
- 15: Eintrittsquerschnitt
- 16: Anschlussbereich
- 17: optisches Fenster
- 18: Pulseletgruppe
- 19: Pulselet
- 20: Puls
- 21: Stimulationsperiode
- 22: Pulsdauer
- 23: Cochlea
- 24: lichtempfindlicher Bereich
- 25: elektrisches Signal
- 26: Hörnerv
- 27: Leiterplatte
- 28: Montageadapter
- 29: Silikonschlauch
- 30: distales Ende

## Patentansprüche

1. Verfahren zum Betreiben eines optischen Cochlea-Implantats (1) mit mehreren Laserdioden (7),
- wobei die verschiedenen Schallfrequenzen zugeordneten Laserdioden (7) abhängig von einem ein Schallfrequenzspektrum beschreibenden Signal angesteuert werden, um Laserlicht (10) zu erzeugen und in verschiedene Lichtleiter (11) einzukoppeln, die dazu vorgesehen sind, das Laserlicht (10) zu verschiedenen lichtempfindlichen Bereichen einer Cochlea zu leiten,
- wobei die Laserdioden (7) jeweils in mehreren voneinander getrennten Pulselets (19), die einzeln keine neurale Aktivierung der lichtempfindlichen Bereiche (24) der Cochlea (23) zur Folge haben, angesteuert werden,
**dadurch gekennzeichnet,**
- **dass** Tastgrade der Pulselets (19) nicht mehr als 25 % betragen und
- **dass** die Pulselets (19), in denen verschiedene der Laserdioden (7) angesteuert werden, zeitlich so ineinander geschachtelt werden, dass nicht mehr als ein Viertel der Laserdioden (7) gleichzeitig angesteuert wird.

2. Verfahren nach Anspruch 1, **dadurch gekennzeichnet,**
- **dass** die Pulselets (19), in denen die einzelnen Laserdioden (7) angesteuert werden, eine Pulseletfolgefrequenz von 5 kHz bis 200 MHz, vorzugsweise von 10 kHz bis 10 MHz, aufweisen und/oder
- **dass** die Tastgrade der Pulselets (19) abhängig von einer Amplitude des Schallfrequenzspektrums bei der Schallfrequenz, der die jeweilige Laserdiode (7) zugeordnet ist, festgelegt werden und vorzugsweise nicht mehr als 12,5 % betragen.

3. Verfahren nach Anspruch 1 oder 2, **dadurch gekennzeichnet, dass** ab einer Mindestamplitude des Schallfrequenzspektrums bei einer Schallfrequenz, der die jeweilige Laserdiode (7) zugeordnet ist, auch solche der Laserdioden (7) angesteuert werden, die benachbarten Schallfrequenzen zugeordnet sind, um das Laserlicht (10) in solche der Lichtleiter (11) einkoppeln, die dazu vorgesehen sind, das Laserlicht (10) zu benachbarten lichtempfindlichen Bereichen (24) der Cochlea (23) zu leiten.

4. Verfahren nach einem der vorhergehenden Ansprüche, **dadurch gekennzeichnet,**
- **dass** die Pulselets (19), in denen eine der Laserdioden (7) angesteuert wird, nacheinander mit den Pulselets (19) geschachtelt werden, in denen verschiedene andere der Laserdioden (7) angesteuert werden, und/oder
- **dass** die Laserdioden (7) abhängig von dem Signal in einer Reihenfolge von Schallfrequenzen angesteuert werden, denen die einzelnen Laserdioden (7) zugeordnet sind, wobei die Schallfrequenzen vorzugsweise in Richtung der Reihenfolge ansteigen.

5. Verfahren nach einem der vorhergehenden Ansprüche, **dadurch gekennzeichnet, dass** die Pulselets (19) in voneinander getrennten effektiven Pulsen, die jeweils eine neurale Aktivierung eines der lichtempfindlichen Bereiche (24) der Cochlea (23) zur Folge haben, mit einer Pulsfolgefrequenz von 50 Hz bis 1.000 Hz gruppiert werden, wobei ein Tastgrad der Pulse nicht mehr als 50 %, vorzugsweise nicht mehr als 25 % beträgt.

6. Verfahren nach einem der vorhergehenden Ansprüche, **dadurch gekennzeichnet, dass** während jedes Pulses nicht mehr als die Hälfte, vorzugsweise nicht mehr als ein Viertel aller Laserdioden (7) angesteuert wird, wobei jeder Puls 2 bis 100, vorzugsweise 5 bis 50 Pulselets (19) je angesteuerter Laserdiode (7) umfasst.

7. Verfahren nach einem der vorhergehenden Ansprüche, **dadurch gekennzeichnet,**
- **dass** nicht mehr als vier, vorzugsweise nicht mehr als zwei der Laserdioden (7) gleichzeitig angesteuert werden, und/oder
- **dass** Diodengruppen von jeweils einigen der Laserdioden (7) getrennt voneinander angesteuert werden, wobei bevorzugt nicht mehr als eine der Laserdioden (7) jeder Diodengruppe gleichzeitig angesteuert wird und wobei optional jede von zwei Diodengruppen 16 und vorzugsweise 32 Laserdioden (7) umfasst.

8. Verfahren nach einem der vorhergehenden Ansprüche, **dadurch gekennzeichnet, dass** die Laserdioden (7) in den Pulselets (19) jeweils mit einem elektrischen Strom
- von mindestens 25 mA, vorzugsweise von mindestens 50 mA und mehr bevorzugt von mindestens 75 mA angesteuert werden.

9. Optisches Cochlea-Implantat (1) mit
- mehreren Laserdioden (7),
- mehreren Lichtleitern (11), die dazu angeordnet und ausgebildet sind, Laserlicht (10) von jeweils einer der Laserdioden (7) in einen von verschiedenen lichtempfindlichen Bereichen einer Cochlea zu leiten,
- einem Energiespeicher (3) und
- einer Steuerung (4), die die verschiedenen Schallfrequenzen zugeordneten Laserdioden (7) abhängig von einem ein Schallfrequenzspektrum beschreibenden Signal mit elektrischen Strömen aus dem Energiespeicher (3) ansteuert, um das Laserlicht (10) zu erzeugen und in die Lichtleiter (11) einzukoppeln,
**dadurch gekennzeichnet, dass** die Steuerung dazu ausgebildet ist, die Laserdioden (7) gemäß dem Verfahren nach einem der vorhergehenden Ansprüche anzusteuern.

10. Optisches Cochlea-Implantat (1) nach Anspruch 9, **gekennzeichnet durch** ein optisches Fenster, über das hinweg sich zumindest einige der Laserdioden (7) und Eintrittsquerschnitte (15) der zugehörigen Lichtleiter (11) gegenüber liegen, wobei zwischen den Laserdioden (7) und dem Fenster (17) ein Mikrolinsenarray (14) angeordnet ist, dessen Mikrolinsen (13) das Laserlicht (10) von jeweils einer der Laserdioden (7) in den Eintrittsquerschnitt des zugehörigen Lichtleiters (11) fokussieren, wobei optische Weglängen zwischen den Austrittflächen der Laserdioden (7) und den Eintrittsquerschnitten der zugehörigen Lichtleiter (11) vorzugsweise nicht länger als 1,5 mm sind.

11. Optisches Cochlea-Implantat (1) mit
- mehreren Laserdioden (7),
- mehreren Lichtleitern (11), die dazu angeordnet und ausgebildet sind, Laserlicht (10) von jeweils einer der Laserdioden (7) in einen von verschiedenen lichtempfindlichen Bereichen (24) einer Cochlea (23) zu leiten, und
- einem optischen Fenster (17), über das hinweg sich zumindest einige der Laserdioden (7) und Eintrittsquerschnitte (15) der zugehörigen Lichtleiter (11) gegenüber liegen,
- wobei zwischen den Laserdioden (7) und dem Fenster (17) ein Mikrolinsenarray (14) angeordnet ist, dessen Mikrolinsen (13) das Laserlicht (10) von jeweils einer der Laserdioden (7) in den Eintrittsquerschnitt (15) des zugehörigen Lichtleiters (11) fokussieren, und
- wobei optische Weglängen zwischen den Austrittflächen der Laserdioden (7) und den Eintrittsquerschnitten (15) der zugehörigen Lichtleiter (11) nicht länger als 1,5 mm ist.

12. Optisches Cochlea-Implantat (1) nach Anspruch 10 oder 11, **dadurch gekennzeichnet,**
- **dass** das optische Fenster (17) eine Dicke von 0,2 mm bis 0,6 mm, vorzugsweise von 0,3 mm bis 0,4 mm aufweist und optional aus Saphir ausgebildet ist, und/oder
- **dass** das Mikrolinsenarray (14) mit einem beidseitig mikrostrukturierten Wafer ausgebildet ist, wobei der Wafer im Bereich zumindest einiger der Mikrolinsen (13) auf zumindest einer seiner Seiten asphärisch strukturiert ist.

13. Optisches Cochlea-Implantat (1) nach einem der Ansprüche 10 bis 12, **dadurch gekennzeichnet, dass** die Laserdioden (7) vor dem optischen Fenster (17) in seitlichen Abständen von 70 µm bis 150 µm, vorzugsweise von 90 µm bis 110 µm angeordnet sind.

14. Optisches Cochlea-Implantat (1) nach einem der Ansprüche 10 bis 13, **dadurch gekennzeichnet, dass** eine der jeweiligen Laserdiode (7) zugekehrte erste Linsenfläche des Mikrolinsenarrays (15) einen Krümmungsradius von 0,060 mm bis 0,075 mm, eine konische Konstante von -2,60 bis -2,50 und eine Brennweite von 0,14 mm bis 0,16 mm aufweist und eine der der jeweiligen Laserdiode (7) abgekehrte zweite Linsenfläche des Mikrolinsenarrays einen Krümmungsradius von 0,120 mm bis 0,130 mm, eine konische Konstante von -0,50 bis -0,40 und eine Brennweite von 0,35 mm bis 0,50 mm aufweist.

15. Optisches Cochlea-Implantat (1) nach einem der Ansprüche 10 bis 14, **dadurch gekennzeichnet,**
- **dass** ein Arbeitsabstand der jeweiligen Laserdiode (7) zu der jeweiligen Mikrolinse (13) zwischen 0,14 mm und 0,16 mm beträgt,
- **dass** eine Linsendicke der jeweiligen Mikrolinse (13) bevorzugt 0,35 mm bis 0,45 mm beträgt,
- **dass** ein Arbeitsabstand der jeweiligen Mikrolinse (13) zu dem optischen Fenster (17) nicht größer als 0,400 mm ist und bevorzugt zwischen 0,09 mm und 0,11 mm beträgt und
- **dass** ein Arbeitsabstand des optischen Fensters (17) zu den Lichtleitern (11) nicht größer als 0,200 mm ist und bevorzugt zwischen 0,09 mm und 0,11 mm beträgt,
- wobei der Arbeitsabstand des optischen Fensters (17) zu den Lichtleitern (11) optional mit transparentem Epoxidharz überbrückt ist.
